# EUROPEAN PATENT APPLICATION

(11) **EP 3 988 109 A2**
(43) Date of publication of application: **27.04.2022**
(21) Application number: 21197536.2
(22) Date of filing: 01.03.2017
(51) Int. Cl.: A61K 38/00, A61K 39/00, A61P 9/00, C07K 7/08, C07K 14/47

(54) **HUMAN MYOSIN PEPTIDES**

(30) Priority: 01.03.2016 GB 201603582
(62) Divisional of application: 17711340.4
(71) Applicant: Apitope International NV, 3590 Diepenbeek (BE)
(72) Inventor: SELLI, Mehmet, Gwent (GB); WRAITH, David, Gwent (GB)
(74) Representative: D Young & Co LLP

(57) **Abstract**

The present invention provides a peptide at least partially derivable from human α-myosin which peptide and the use of such peptides for the prevention or suppression of autoantibody formation in myocarditis.

## Description

### FIELD OF THE INVENTION

### BACKGROUND TO THE INVENTION

Myocarditis is the leading cause of heart failure in people under 40 years of age. Up to one fifth of those affected by an acute episode will progress towards chronic disease leading to dilated cardiomyopathy (DCM). Acute myocarditis can result from infection by viruses including coxsackevirus B3 (CVB3); evidence of prior infection is found in approximately 50% of patients with DCM. Viruses such as CVB3 have a direct cytopathic effect on cardiac tissue. Strong evidence suggests, however, that individuals who progress towards chronic myocarditis do so because they develop an autoimmune response to antigens of cardiac muscle.

Circulating antibodies to cardiac tissue antigens, including myosin, are associated with chronic myocarditis and the severity of cardiac disease is directly related to levels of autoantibodies in the affected tissue. Organ specific IgG antibodies were found in biopsies from patients with DCM. B cells secreting IgG depend on CD4⁺ T cell help for class switching and it is, therefore, not surprising that T-cells specific for cardiac muscle are found in biopsies of patients with myocarditis. T cells isolated from patients with myocarditis have been shown to induce disease in SCID mice.

Myosin alpha chain is specific to cardiac muscle whereas the beta chain is also expressed in skeletal muscle. Among DCM patients, 41% were shown to have antibodies to α-myosin while 20% of direct relatives also had similar antibodies. This suggests either a genetic or environmental contribution to disease susceptibility. There remains the possibility that some cases of myocarditis may have an autoimmune origin without necessarily following viral damage to heart tissue. This is supported by clinical evidence that patients who have no evidence of viral genome in their heart tissue can be treated with immunosuppressive drugs.

In general, treatment of either acute or chronic myocarditis is aimed at reducing congestion and improving cardiac hemodynamics in heart failure. Treatment of heart failure follows the same treatment regimen regardless of the underlying cause (i.e., vasodilators, diuretics, beta-adrenergic blockers, angiotensin-converting enzyme inhibitors).

There is currently no cure available for chronic myocarditis and present treatments are therefore directed towards targeting the presenting symptoms. There is thus a need for alternative therapies for myocarditis that are effective at treating or preventing myocarditis and at alleviating or reducing the symptoms of the disease.

The present invention addresses this need.

### SUMMARY OF ASPECTS OF THE INVENTION

As shown in the present Examples, the present inventors have identified a number of peptides derived from α-myosin which are effective in reducing autoantibodies to α-myosin, reducing the proliferation of T cells specific for cardiac myosin, and improving left ventricular cardiac function. The peptides of the invention are useful in the prevention and/or treatment of autoimmune myocarditis.

Thus in a first aspect, the present invention provides a polypeptide comprising:
(i) all or a part of an amino acid sequence selected from:
   MLTFLHEPAVLFNLK (SEQ ID NO:1),
   VNPYKWLPVYNAEVV (SEQ ID NO:2),
   PHIFSISDNAYQYML (SEQ ID NO:3),
   KRVIQYFASIAAIGD (SEQ ID NO:4),
   YHIFYQILSNKKPEL (SEQ ID NO:5),
   KSAYLMGLNSADLLK (SEQ ID NO:6),
   KSSLKLMATLFSSYA (SEQ ID NO:7),
   KGSSFQTVSALHREN (SEQ ID NO:8),
   EATLQHEATAAALRK (SEQ ID NO:9) and
   RVQLLHSQNTSLINQ (SEQ ID NO:10); or
(ii) an amino acid sequence having at least 60% sequence identity to the sequences of any of SEQ ID NO:1 to 10.

### DESCRIPTION OF THE FIGURES

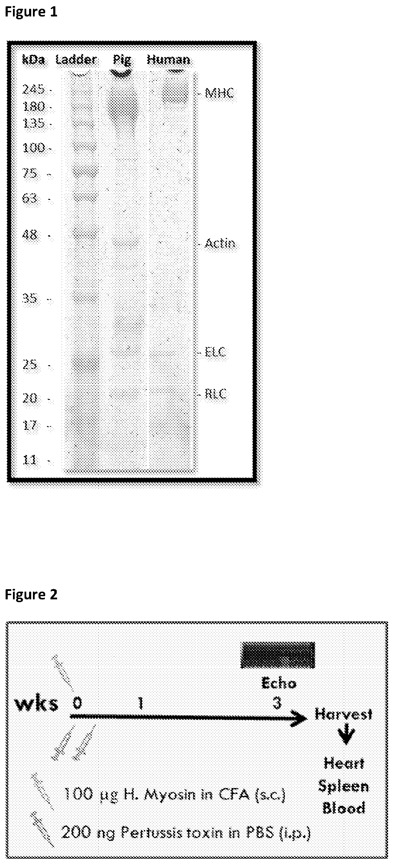

**Figure 1** shows the SDS gel image of human and pig cardiac myosin extracts stained with Coomassie blue. Pig cardiac myosin is a crude extraction without the final actin and actinomyosin removal steps. Human cardiac myosin is purer giving only the myosin heavy chain (MyHC), essential light chain (ELC) and the regulatory light chain (RLC) bands.
**Figure 2** shows the protocol for experimental autoimmune myocarditis (EAM) induction in DR4 mice. 100 µg of human myosin extract emulsified in CFA was subcutaneously injected into mice at 6-8 weeks of age. Two peritoneal injections of 200 ng Pertussis toxin were also given on day 0 and 2. The development of the disease was assessed after 3 weeks.

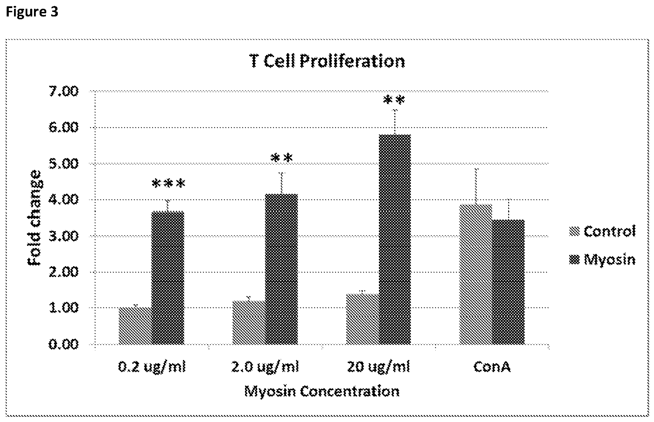

**Figure 3** shows the increased T cell proliferation against cardiac myosin in the EAM model.
   Cardiac myosin injection increased the anti-myosin T cell proliferation (p=0.00015 for 0.2 µg/ml, p=0.0037 for 2.0 µg/ml and p=0.0015 for 20 µg/ml myosin concentration; n=5), even more than the global T cell stimulator, ConA, did at a final concentration of 2 µg/ml. (A two tailed unpaired student t-test was used to determine the differences between the means. When comparing more than two groups of means, a one-way ANOVA was performed followed by a Bonferroni correction test, to assess significant differences amongst groups.)

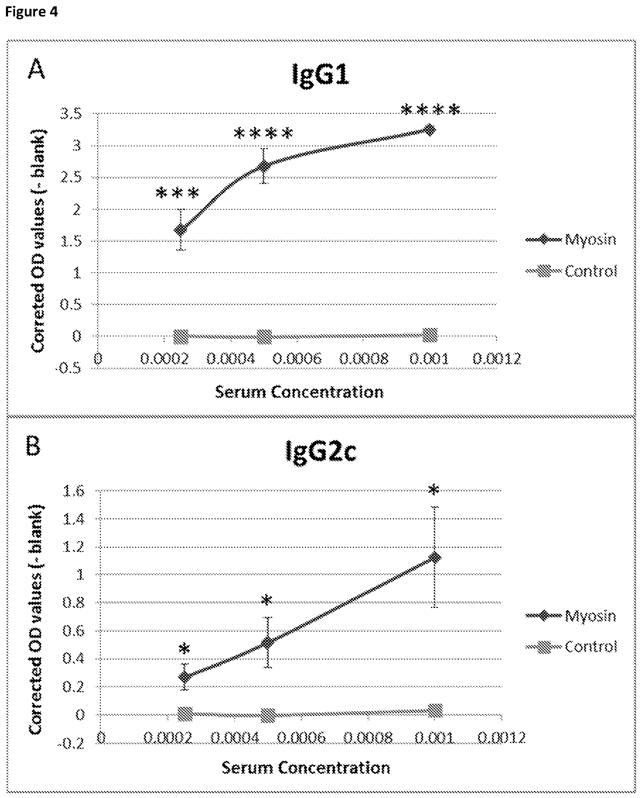

**Figure 4** shows the increased anti-myosin antibody levels in EAM induced DR4 mice **(4A)** Circulating IgG1 levels were increased significantly in the group immunized with α-myosin (pₘₐₓ=0.0008; n=5) **(4B)** IgG2c levels were significantly increased in the EAM model too (pₘₐₓ=0.02; n=5)

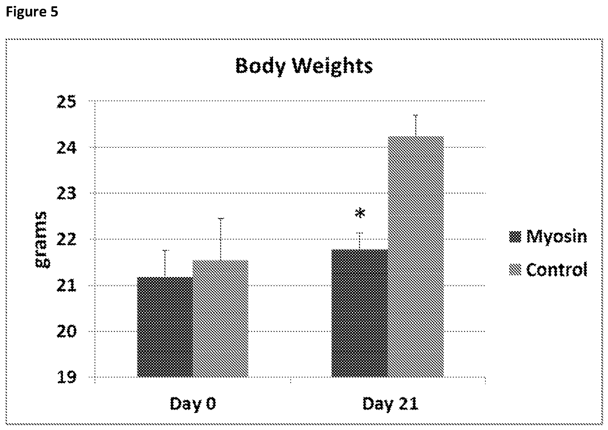

**Figure 5** shows significant decrease in body weights among mice. The myosin inoculated group gained only half a gram on average, while the control group gained almost 3 grams in 3 weeks. The difference in body weight was significant at the end (p=0.04; n=5)

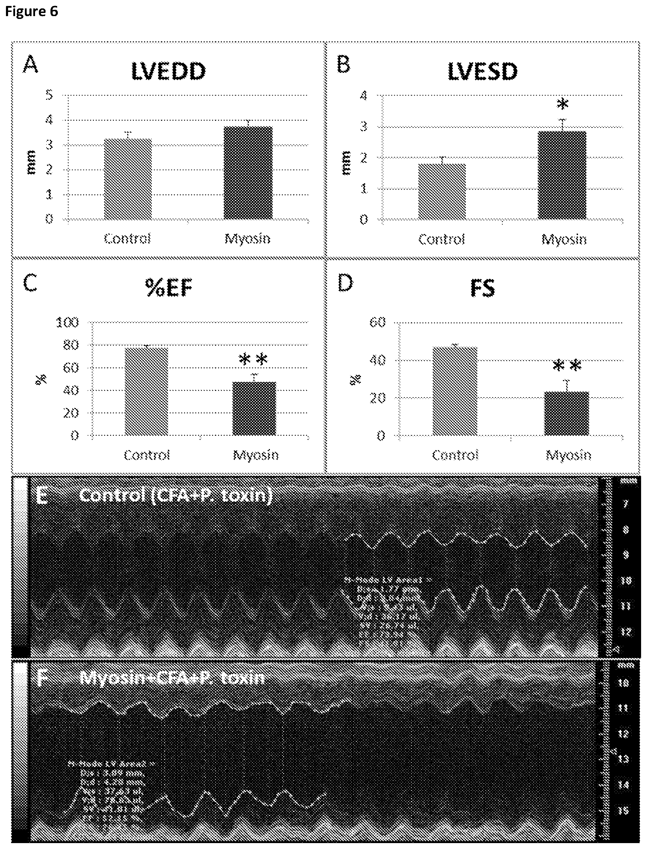

**Figure 6** shows impaired left ventricular cardiac function in EAM induced DR4 mice. (A) No significant difference was noted between left ventricle-end-diastolic diameters (B) A significant increase in left ventricle-end-systolic diameter was observed (p=0.039; n=5) (C) A significant reduction in the percentage of ejection fraction occurred (p=0.003; n=5) (D) A significant reduction in the percentage of fractional shortening (p=0.006; n=5) was also found (E) A representative image is shown for the control group taken in M-mode (F) A representative image for the EAM induced group taken in M-mode

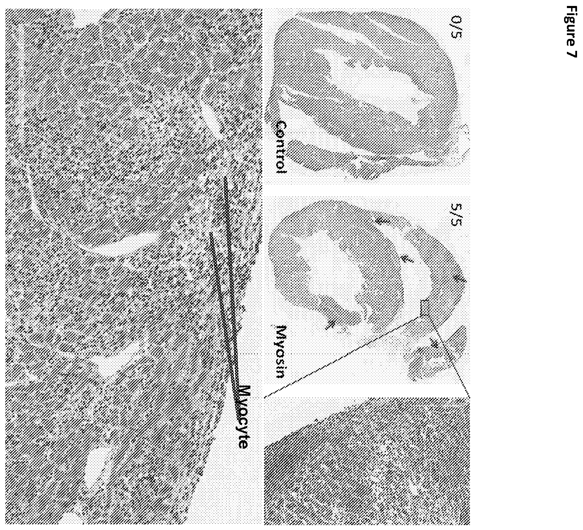

**Figure 7** shows representative H&E sections demonstrating the inflamed areas on hearts with myocarditis. The mice in the control group were all clear, while 5/5 of the hearts from EAM induced mice were infiltrated with immune cells (p=0.0079, Fisher's exact test).

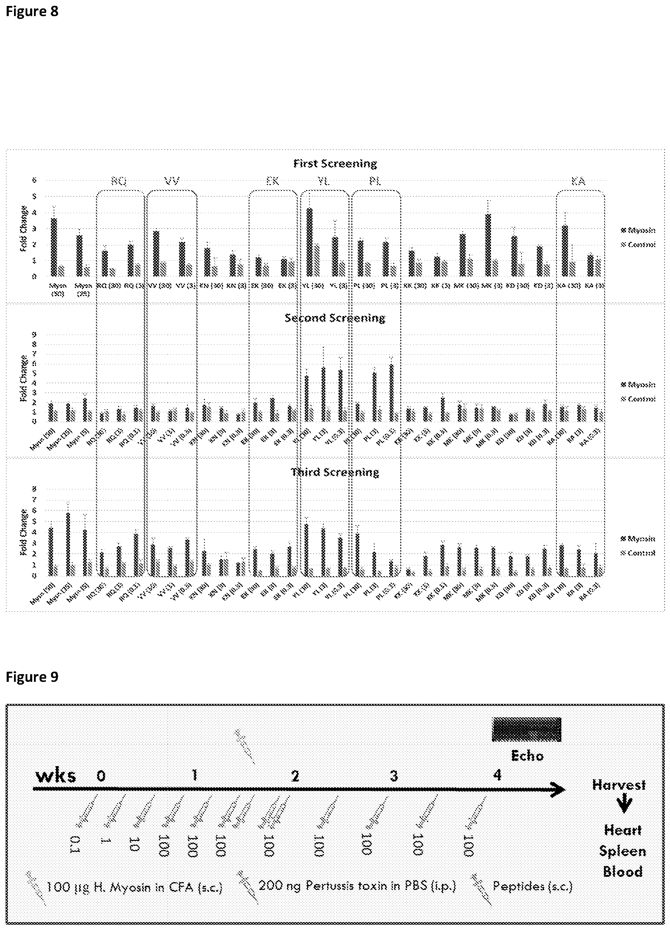

**Figure 8** shows the screening of the candidate epitopes for their antigenicity. The screening assay were repeated three times and the dominant epitopes were determined according to their stimulation index. The number in brackets refers to the concentration of the peptides or α-myosin (µg/ml) used to stimulate the cells
**Figure 9** shows the α-myosin specific peptide therapy protocol.

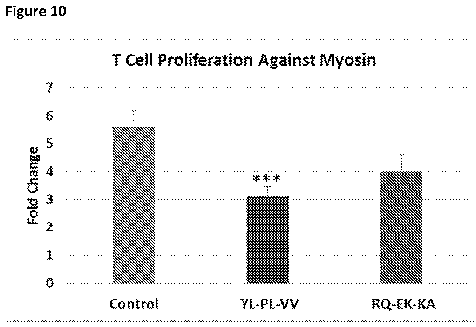

**Figure 10** shows decreased anti-myosin T cell proliferation obtained by the peptide therapies. YL-PL-VV peptides (peptides 5, 3 and 2) induced a significant decrease (p=0.0007; n=15), while RQ-EK-KA peptides (peptides 10, 9 and 7) provided a decreasing trend without statistical significance (p=0.065; n=11).

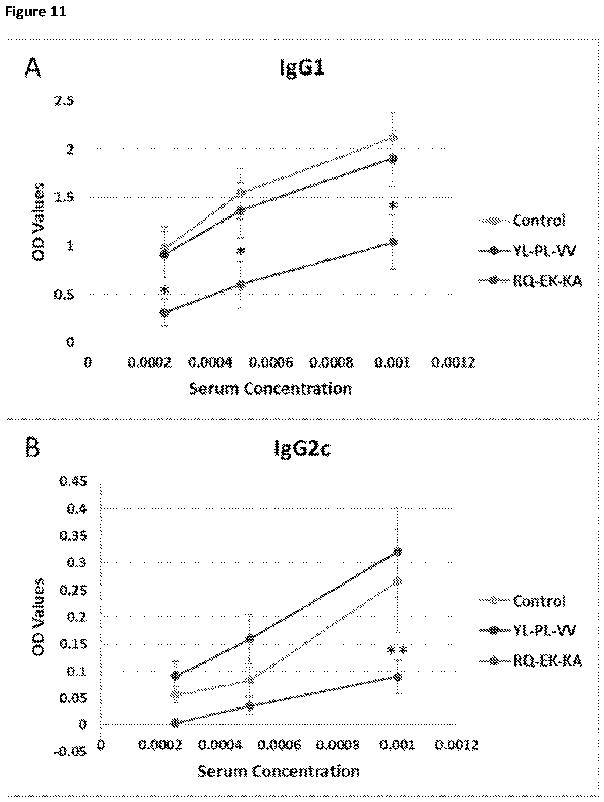

**Figure 11** shows decreasing myosin-specific antibody levels induced by RQ-EK-KA therapy. (A) Anti-myosin IgG1 levels were decreased significantly with RQ-EK-KA peptides, compared to the control group, at all serum concentrations (pₘₐₓ=0.036; n=11). (B) Anti-myosin IgG2c levels were decreased significantly with RQ-EK-KA peptides at 1/1000 dilution (p=0.0072; n=11).

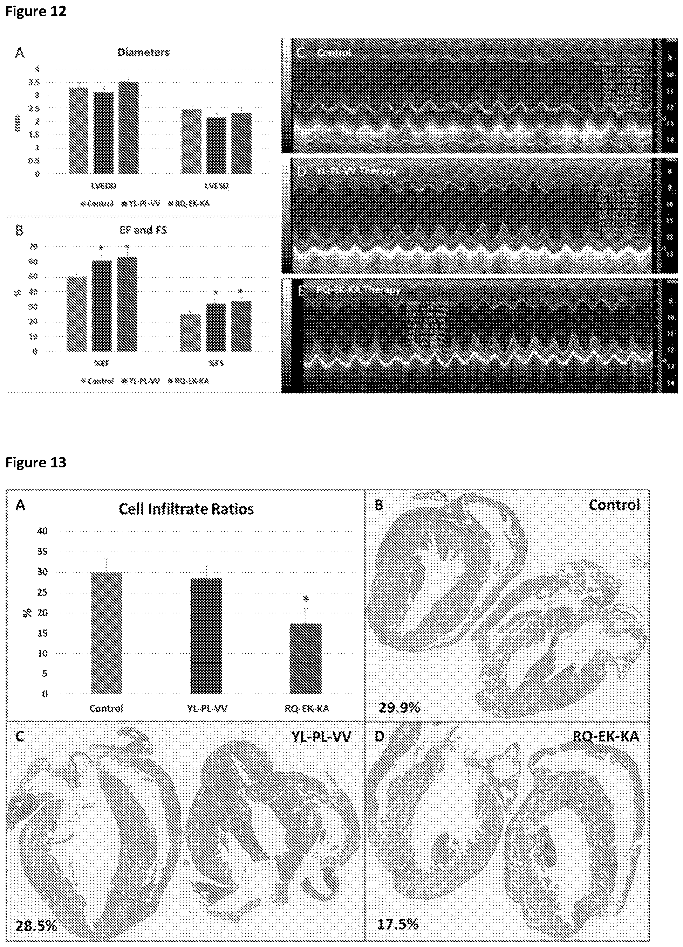

**Figure 12** shows the improved left ventricular cardiac function obtained by α-myosin-specific peptide therapies. (A) Left ventricle-end-diastolic and systolic dimensions were not affected by peptide therapies (B) Significant increases in the percentage of ejection fraction (p=0.011, n=10 for RQ-EK-KA therapy; p=0.041, n=13 for YL-PL-VV therapy) and fractional shortening (p=0.011, n=10 for RQ-EK-KA therapy; p=0.044, n=13 for YL-PL-VV therapy) were achieved by both therapies (C) A representative image for the control group (no therapy) taken in M-mode (D) A representative image for the group receiving YL-PL-VV therapy (E) A representative image for the group receiving RQ-EK-KA therapy
**Figure 13** shows decreased inflammatory cell infiltration into cardiac tissue obtained by RQ-EK-KA therapy. (A) The level of cardiac inflammation identified by a cell infiltrate was reduced significantly by RQ-EK-KA therapy (p=0.021; n=11), whereas YL-PL-VV therapy had no effect. Representative H&E sections showing the ratio of cell infiltrate (blue areas due to concentrated cell nuclei stained with hematoxylin) from (B) control (C) YL-PL-VV therapy and (D) RQ-EK-KA therapy groups.

### DETAILED DESCRIPTION

### PEPTlDES

In a first aspect, the present invention relates to a peptide.

The term "peptide" is used in the normal sense to mean a series of residues, typically L-amino acids, connected one to the other, typically by peptide bonds between the α-amino and carboxyl groups of adjacent amino acids. The term includes modified peptides and synthetic peptide analogues.

The peptide of the present invention may be made using chemical methods (Peptide Chemistry, A practical Textbook. Mikos Bodansky, Springer-Verlag, Berlin.). For example, peptides can be synthesized by solid phase techniques (Roberge JY et al (1995) Science 269: 202-204), cleaved from the resin, and purified by preparative high performance liquid chromatography (e.g., Creighton (1983) Proteins Structures And Molecular Principles, WH Freeman and Co, New York NY). Automated synthesis may be achieved, for example, using the ABI 43 1 A Peptide Synthesizer (Perkin Elmer) in accordance with the instructions provided by the manufacturer.

The peptide may alternatively be made by recombinant means, or by cleavage from a longer polypeptide. For example, the peptide may be obtained by cleavage from the α myosin protein, which may be followed by modification of one or both ends. The composition of a peptide may be confirmed by amino acid analysis or sequencing (e.g., the Edman degradation procedure).

For practical purposes, there are various other characteristics which the peptide may show. For example, it is important that the peptide is sufficiently stable *in vivo* to be therapeutically useful. The half-life of the peptide *in vivo* may be at least 10 minutes, 30 minutes, 4 hours, or 24 hours.

The peptide may also demonstrate good bioavailability *in vivo.* The peptide may maintain a conformation *in vivo* which enables it to bind to an MHC molecule at the cell surface without due hindrance.

The peptide according to the present invention may comprise or consist of an amino acid sequence having at least 60%, 61%, 62%, 63%, 64%, 65%, 66%, 67%, 68%, 69%, 70%, 71%, 72%, 73%, 74%, 75%, 76%, 77%, 78%, 79%, 70%, 81%, 82%, 83%, 84%, 85%, 86%, 87%, 88%, 89%, 90%, 91%, 92%, 93%, 94%, 95%, 96%, 97%, 98%, 99% or 100% identity with a peptide selected from any one of SEQ ID NOs: 1 to 10. In one aspect the peptide has at least 70%, 75%, 80%, 85%, 90%, 91%, 92%, 93%, 94%, 95%, 96%, 97%, 98%, 99% or 100% sequence identity to any one of SEQ ID NOs:1 to 10.

In a preferred aspect the peptide comprises any one of SEQ ID NOs:1 to 10. In a further preferred aspect the peptide consists of any one of SEQ ID NOs:1 to 10, preferably SEQ ID NO: 2, 3, 5, 7, 9 or 10.

Sequence identity may be assessed by any convenient method. However, for determining the degree of sequence identity between sequences, computer programs that make multiple alignments of sequences are useful, for instance Clustal W (Thompson et al., (1994) Nucleic Acids Res., 22: 4673-4680). Programs that compare and align pairs of sequences, like ALIGN (Myers ef a/., (1988) CABIOS, 4: 1-17), FASTA (Pearson er a/., (1988) PNAS, 85:2444-2448; Pearson (1990), Methods Enzymol., 183: 63-98) and gapped BLAST (Altschul er a/., (1997) Nucleic Acids Res., 25: 3389-3402) are also useful for this purpose. Furthermore, the Dali server at the European Bioinformatics institute offers structure-based alignments of protein sequences (Holm (1993) J. Mol. Biol., 233: 123-38; Holm (1995) Trends Biochem. Sci., 20: 478-480; Holm (1998) Nucleic Acid Res., 26: 316-9).

Multiple sequence alignments and percent identity calculations may be determined using the standard BLAST parameters, (using sequences from all organisms available, matrix Blosum 62, gap costs: existence 11, extension 1).

Alternatively, the following program and parameters may be used: Program: Align Plus 4, version 4.10 (Sci Ed Central Clone Manager Professional Suite). DNA comparison: Global comparison, Standard Linear Scoring matrix, Mismatch penalty = 2, Open gap penalty = 4, Extend gap penalty = 1. Amino acid comparison: Global comparison, BLOSUM 62 Scoring matrix.

Thus included in the scope of the invention are variants of the stated or given sequences, as long as the variant retains the functional activity of the parent i.e. the variants are functionally equivalent, in other words they have or exhibit an activity of the parent peptide as defined herein (e.g a beneficial effect in myocarditis). Such variants may comprise amino acid substitutions, additions or deletions (including truncations at one or both ends) of the parent sequence e.g. of one or more e.g. 1 to 14 amino acids.

Also included are functionally-equivalent derivatives in which one or more of the amino acids are chemically derivatised, e.g. substituted with a chemical group.

Thus, the peptides of the invention can comprise parts or fragments of SEQ ID NOs:1-10, provided that the peptide retains the required activity. Fragments of SEQ ID NOs:1-10 may for example be from 7 to 14 residues in length, e.g. 7, 8, 9, 10, 11, 12 or 13 residues in length.

In a further aspect, the invention provides a nucleic acid molecule encoding a peptide as defined herein, namely a peptide having or comprising:
(i) all or a part of an amino acid sequence selected from:
   MLTFLHEPAVLFNLK (SEQ ID NO:1),
   VNPYKWLPVYNAEVV (SEQ ID NO:2),
   PHIFSISDNAYQYML (SEQ ID NO:3),
   KRVIQYFASIAAIGD (SEQ ID NO:4),
   YHIFYQILSNKKPEL (SEQ ID NO:5),
   KSAYLMGLNSADLLK (SEQ ID NO:6),
   KSSLKLMATLFSSYA (SEQ ID NO:7),
   KGSSFQTVSALHREN (SEQ ID NO:8),
   EATLQHEATAAALRK (SEQ ID NO:9) and
   RVQLLHSQNTSLINQ (SEQ ID NO:10); or
(ii) an amino acid sequence having at least 60% sequence identity to the sequences of any of SEQ ID NO:1 to 10.

Also provided is the complement of such a nucleic acid molecule.

The nucleic acid molecule of the invention preferably comprises at least 30 nucleotides and preferably no more than 800 nucleotides, more preferably no more than 700, 650, 600, 550, 500, 450, 400, 350, 300, 250, 200, 150, 100 or 50 nucleotides. The nucleic acid molecule is preferably an isolated molecule.

### EPITOPES

In an adaptive immune response, T lymphocytes are capable of recognising internal epitopes of a protein antigen. Antigen presenting cells (APC) take up protein antigens and degrade them into short peptide fragments. A peptide may bind to a major histocompatibility complex (MHC) molecule inside the cell and be carried to the cell surface. When presented at the cell surface in conjunction with an MHC molecule, the peptide may be recognised by a T cell (via the T cell receptor (TCR)), in which case the peptide is a T cell epitope.

An epitope is thus a peptide derivable from an antigen which is capable of binding to the peptide-binding groove of an MHC molecule and being recognised by a T cell. The minimal epitope is the shortest fragment derivable from an epitope, which is capable of binding to the peptide-binding grove of an MHC molecule and being recognised by a T cell.

A peptide of the present invention may be any length that is capable of binding to an MHC molecule. Typically, the peptide of the present invention is capable of binding MHC class II.

Peptides that bind to MHC class I molecules are typically 7 to 13, more usually 8 to 10 amino acids in length. The binding of the peptide is stabilised at its two ends by contacts between atoms in the main chain of the peptide and invariant sites in the peptide-binding groove of all MHC class I molecules. There are invariant sites at both ends of the groove which bind the amino and carboxy termini of the peptide. Variations in peptide length are accommodated by a kinking in the peptide backbone, often at proline or glycine residues that allow flexibility.

Peptides which bind to MHC class II molecules are typically between 8 and 20 amino acids in length, more usually between 10 and 17 amino acids in length, and can be longer (for example up to 40 amino acids). These peptides lie in an extended conformation along the MHC II peptide-binding groove which (unlike the MHC class I peptide-binding groove) is open at both ends. The peptide is held in place mainly by main-chain atom contacts with conserved residues that line the peptide-binding groove.

The peptide of the present invention may comprise between 8 and 30 amino acids, for example 8 to 25 amino acids, 8 to 20 amino acids, 8 to 15 amino acids or 8 to 12 amino acids. In one aspect the peptide of the present invention may thus be 8, 9, 10, 11, 12, 13, 14, 15, 16, 17, 18, 19, 20, 21, 22, 23, 24, 25, 26, 27, 28, 29 or 30 amino acids in length. In one embodiment the peptide is 20, 21 or 22 amino acids in length.

A peptide according to the present invention may comprise a modification relative to any one of SEQ ID NOs:1 to 10. For example, such a peptide may comprise one or more modifications such as mutations, insertions, deletions, additions and/or substitutions, providing the peptide retains its function as described herein. Typically, the modification may be amino acid substitutions within the α-myosin derived sequence. The amino acid may be substituted for an amino acid such as glycine, lysine or glutamic acid. The peptide may comprise up to three, up to two or one amino acid substitution from the α-myosin derived sequence.

Such a peptide may comprise amino acids at one or both ends which are not derivable from the α-myosin sequence. For example, the peptide may have one or more glycine and/or lysine and/or glutamic acid residues at one or both ends. For example, the additional amino acids may comprise a glycine or lysine spacer, followed by the amino acid pairs KK, KE, EK or EE at one or both ends

In one embodiment the peptide may comprise any amino acid that may improve or optimise the druggability of the peptide, for example natural or artificial amino acids can improve solubility of peptides.

For example, the peptide may have the following formula:
KKG- α-myosin -derived peptide-GKK

### α-MYOSIN

The terms α-myosin and Myosin Heavy Chain (MyHC)-α are used herein synonymously.

Chronic myocarditis is an autoimmune disease caused by auto-reactive T and B lymphocytes targeting cardiac autoantigens, in particular α-myosin.

Cardiac muscle myosin is a hexamer consisting of two heavy chain subunits, two light chain subunits, and two regulatory subunits. The heavy chain is made up of α (alpha) heavy chain subunit (MyHC-α) and β (beta) heavy chain subunits (MyHC-β).

Changes in the relative abundance of MyHC-β and MyHC-a correlate with the contractile velocity of cardiac muscle. In early foetal development, MyHC-β is predominately expressed in the ventricles, while MyHC-α is predominantly expressed in the atria. In healthy adult hearts, MyHC-α is predominantly expressed in the atria (>90%), while MyHC-β (~50%) is expressed in the atria of failing adult hearts. *In vitro* studies have demonstrated that newly formed cardiac myocytes express MyHC-β, and after prolonged (1-5 weeks) contractile activity MyHC-α becomes detectable. An allelic variant of this gene is associated with approximately 40% of the cases of Hypertrophic cardiomyopathy (HCM).

As discussed above, myocarditis is characterised by an infection of the heart with an inflammatory infiltrate and damage to the heart muscle, without the blockage of coronary arteries that define a myocardial infarction or other common noninfectious causes. Myocarditis is the leading cause of heart failure in people under forty years of age. The standard Dallas pathological criteria for the definition of myocarditis require that an inflammatory cellular infiltrate with or without associated myocyte necrosis be present on conventionally stained heart-tissue sections (Cooper et al.; N Engl J Med; 2009; 260:1526-1538).

Acute phase myocarditis is usually caused by viral cardiac damage, most often due to infection by common viruses, such as parvovirus B19, and less commonly nonviral pathogens such as Borrelia burgdorferi (Lyme disease) or Trypanosoma cruzi, or as a hypersensitivity response to drugs.

Approximately one third of cases of acute myocarditis resolve, but dilated cardiomyopathy (DCM) with chronic congestive heart failure is the major long-term outcome of myocarditis (Cooper et al.; as above). DCM is a condition in which the heart becomes weakened and enlarged and cannot pump blood efficiently. The decreased heart function can affect the lungs, liver, and other body systems.

Chronic autoimmunity is believed to underlie the progression from myocarditis to DCM. Strong T and B cell responses to cardiac myosin, in particular α-myosin, may arise through molecular mimicry of pathogenic (e.g. viral) antigens or due to the activation of autoreactive immune cells present upon tissue damage associated exposure of internal cardiac myosin because the thymus does not express cardiac myosin.

The amino acid sequence of human α-myosin (MyHC-α) is given below (SEQ ID No. 11).

 60
 120
 180
 240
 300
 360
 420
 480
 540
 600
 660
 720
 780
 840
 900
 960
 1020
 1080
 1140
 1200
 1260
 1320
 1380
 1440
 1500
 1560
 1620
 1680
 1740
 1800
 1860
 1920
 1939

The peptide of the invention may be at least partially derivable from α-myosin.

### TOLERANCE

Tolerance is state of unresponsiveness to an antigen. Tolerance to self antigens is an essential feature of the immune system, when this is lost, autoimmune disease can result. The adaptive immune system must maintain the capacity to respond to an enormous variety of infectious agents while avoiding autoimmune attack of the self antigens contained within its own tissues. This is controlled to a large extent by the sensitivity of immature T lymphocytes to apoptotic cell death in the thymus (central tolerance). However, not all self antigens are detected in the thymus, so death of self-reactive thymocytes remains incomplete. There are thus also mechanisms by which tolerance may be acquired by mature self-reactive T lymphocytes in the peripheral tissues (peripheral tolerance). A review of the mechanisms of central and peripheral tolerance is given in Anderton et al (1999) (Immunological Reviews 169:123-137).

Chronic myocarditis is currently believed to be caused by anti-α-myosin autoantibodies. As demonstrated herein, peptides according to the present invention are capable of inducing tolerance to α-myosin, such that when administered to a subject, they may reinstate tolerance to the α-myosin self-protein and curtail the pathogenic immune response.

Tolerance may result from or be characterised by the induction of anergy in at least a portion of CD4+ T cells. In order to activate a T cell, a peptide must associate with a "professional" APC capable of delivering two signals to T cells. The first signal (signal 1) is delivered by the MHC-peptide complex on the cell surface of the APC and is received by the T cell via the TCR. The second signal (signal 2) is delivered by costimulatory molecules on the surface of the APC, such as CD80 and CD86, and received by CD28 on the surface of the T cell. It is thought that when a T cell receives signal 1 in the absence of signal 2, it is not activated and, in fact, becomes anergic. Anergic T cells are refractory to subsequent antigenic challenge, and may be capable of suppressing other immune responses (T reg cells). Anergic T cells/Treg cells are thought to be involved in mediating T cell tolerance.

The induction of T cell tolerance to α-myosin can be monitored in vivo by looking for a reduction in the level of:
i) α-myosin autoantibodies;
ii) CD4+ T cells specific for α-myosin; and/or
iii) B cells capable of secreting α-myosin autoantibodies
by techniques known in the art.

The induction of tolerance can therefore also be monitored by various techniques including:
(a) the induction of anergy/Treg in CD4+ T cells (which can be detected by subsequent challenge with antigen *in vitro*);
(b) changes in the CD4+ T cell population, including
   (i) reduction in proliferation;
   (ii) down-regulation in the production of IL-2, IFN-γ and IL-4; and
   (iii) increase in the production of IL-10.

As used herein, the term "tolerogenic" means capable of inducing tolerance, i.e. antigen-specific immune tolerance.

### COMPOSITION

The present invention also relates to a composition, such as a pharmaceutical composition comprising one or more peptide(s) according to the invention.

The composition may comprise a plurality of peptides, for example two, three, four, five or six peptides.

In a preferred aspect the composition comprises two or more different peptides, for example two, three, four, five, six, seven, eight, nine or ten or more different peptides. In a preferred aspect the composition comprises three different peptides, preferably peptides of SEQ ID NOs: 2, 3 and 5, or 7, 9 and 10.

The composition of the present invention may be for prophylactic or therapeutic use.

When administered for prophylactic use, the composition may reduce or prevent the generation of an immune response to α-myosin. The level of immune response is less than would be obtained if the patient had not been treated the composition. The term "reduce" indicates that a partial reduction in immune response is observed, such as a 50%, 70%, 80% or 90% reduction in the response that would have been observed if the patient had not been treated with the composition (or in the response observed in an untreated patient over the same time-period). The term "prevent" indicates that no appreciable immune response to α-myosin is observed.

The subject may be any human or non-human animal subject, but more particularly may be a mammal. The animal may be a livestock or a domestic animal or an animal of commercial value, including laboratory animals or an animal in a zoo or game park. Representative animals therefore include dogs, cats, rabbits, mice, guinea pigs, hamsters, horses, pigs, sheep, goats, cows, chickens, turkeys, guinea fowl, ducks, geese, parrots, budgerigars, pigeons. Veterinary uses of the invention are thus covered. The subject may be viewed as a patient. Preferably the subject is a human.

When administered for therapeutic use, the composition may suppress an already on-going immune response to α-myosin. The term "suppress" indicates a reduction in the level of an on-going immune response, compared to the level before peptide treatment, or the levels which would have been observed at the same time point had the treatment not been given.

Treatment with the composition of the present invention may cause a reduction in level of any or all of the following:
i) α-myosin autoantibodies;
ii) CD4+ T cells specific for α-myosin; and
iii) B cells secreting α-myosin autoantibodies.

Detection of all of the factors can be carried out by techniques known in the art, such as ELISA, flow cytometry etc.

Treatment with the composition of the present invention may also or alternatively cause anergy in CD4+ T cells or induce Treg cells specific for α-myosin. Anergy can be detected by, for example, subsequent challenge with α-myosin *in vitro.*

Where there are two or more peptides, the pharmaceutical composition may be in the form of a kit, in which some or each of the peptides are provided separately for simultaneous, separate or sequential administration.

Alternatively (or in addition) if the pharmaceutical composition (or any part thereof) is to be administered in multiple doses, each dose may be packaged separately.

Also, in the pharmaceutical compositions of the present invention, the, or each, peptide may be admixed with any suitable binder(s), lubricant(s), suspending agent(s), coating agent(s), or solubilising agent(s).

### FORMULATION

The composition may by prepared as an injectable, either as liquid solution or suspension; solid form suitable for solution in, or suspension in, liquid prior to injection may also be prepared. The preparation may also be emulsified, or the peptides encapsulated in liposomes. The active ingredients may be mixed with excipients which are pharmaceutically acceptable and compatible with the active ingredient. Suitable excipients are, for example, water, saline (for example, phosphate-buffered saline), dextrose, glycerol, ethanol, or the like and combinations thereof.

In addition, if desired, the composition may contain minor amounts of auxiliary substances such as wetting or emulsifying agents and/or pH buffering agents. Buffering salts include phosphate, citrate, acetate, hydrochloric acid and/or sodium hydroxide may be used for pH adjustment. For stabilisation, disaccharides may be used such as sucrose or trehalose.

If the composition comprises a plurality of peptides, the relative ratio of the peptides may be approximately equal. Alternatively the relative ratios of each peptide may be altered, for example, to focus the tolerogenic response on a particular sub-set of autoreactive T-cells or if it is found that one peptide works better than the others in particular HLA types.

After formulation, the composition may be incorporated into a sterile container which is then sealed and stored at a low temperature, for example 4°C, or it may be freeze-dried. Conveniently the composition is prepared as a lyophilized (freeze dried) powder. Lyophilisation permits long-term storage in a stabilised form. Lyophilisation procedures are well known in the art, see for example http://www.devicelink.com/ivdt/archive/97/01/006.html. Bulking agents are commonly used prior to freeze-drying, such as mannitol, dextran or glycine.

The pharmaceutical composition may be provided in any form known in the art, for example as a tablet, capsule, coated tablet, liquid, suspension, tab, sachet, implant, inhalant, powder, pellet, emulsion, lyophilisate, effervescent, spray, salve, emulsion, balm, plaster or any mixtures thereof. It may be provided e.g. as a gastric fluid-resistant preparation and/or in sustained action form. It may be a form suitable for oral, parenteral, topical, rectal, genital, subcutaneous, transurethral, transdermal, intranasal, intraperitoneal, intramuscular and/or intravenous administration and/or for administration by inhalation.

The composition may be administered in a convenient manner such as by the oral, intravenous (where water soluble), intramuscular, subcutaneous, sublingual, intranasal, intradermal or suppository routes or implanting (e.g. using slow release molecules).

The composition may advantageously be administered via intranasal, subcutaneous or intradermal routes.

The peptide and composition of the invention may be used to treat a human subject. The subject may have myocarditis. The subject may have chronic autoimmune myocarditis. The subject may have dilated cardiomyopathy. The subject may have α-myosin autoantibodies.

The subject may be an HLA-haplotype which is associated with a predisposition to develop α-myosin autoantibodies. The subject may express HLA-DR4. Methods for determining the HLA haplotype of an individual are known in the art.

Typically, a physician will determine the actual dosage which will be most suitable for an individual subject and it will vary with the age, weight and response of the particular patient.

In a preferred embodiment a "dose escalation" protocol may be followed, where a plurality of doses is given to the patient in ascending concentrations. Such an approach has been used, for example, for phospholipase A2 peptides in immunotherapeutic applications against bee venom allergy (Müller et al (1998) J. Allergy Clin Immunol. 101:747-754 and Akdis et al (1998) J. Clin. Invest. 102:98-106).

### KITS

Conveniently, if the composition comprises a plurality of peptides, they may be administered together, in the form of a mixed composition or cocktail. However, there may be circumstances in which it is preferable to provide the peptides separately in the form of a kit, for simultaneous, separate, sequential or combined administration.

The kit may also comprise mixing and/or administration means (for example a vapouriser for intranasal administration; or a syringe and needle for subcutaneous/intradermal dosing). The kit may also comprise instructions for use.

The pharmaceutical composition or kit of the invention may be used to treat and/or prevent a disease.

In particular, the composition/kit may be used to treat and/or prevent chronic myocarditis.

### EXPERIMENTAL ANIMAL MODEL

As described in the Examples section, the inventors have successfully developed and used an experimental model for use in studying experimental autoimmune myocarditis (EAM), and hence which is useful in the study of myocarditis.

As such, in one aspect the invention provides an animal model of EAM. The animal may be a mammal. For example the animal may be a mouse, rat, rabbit, guinea pig or primate. Preferably, the animal is a mouse.

The mouse model of EAM comprises an HLA-DR4tg mouse which has been immunised with cardiac myosin, preferably cardiac α-myosin, more preferably human cardiac α-myosin. The DR4 mouse strain was originally created by Lars Fugger et al (PNAS 1994 volume 91: 6151-55) in that a HLA-DRA*01:01/ HLA-DRB1*04:01 and mCD3-huCD4c/g constructs were co-microinjected into embryos from (DBA/1xA/CA) F1 matings and viable embryos were transferred into pseudopregnant female (BALB/c x 129) F1 for development to term. The offspring has later been bred into the IA-b knockout C57BL/6 genetic background (AB⁰ mice) lacking mouse MHC class II molecule expression. The only MHC class II molecule expressed in these DR4 mice is therefore the human HLA DR4 molecule. One skilled in the art will understand how to obtain such mice.

Suitable immunisation methods will be known to those skilled in the art, for example as described in the present Examples. Sources of cardiac myosin are known to one skilled in the art, for example it may be extracted from human cardiac tissue by methods known in the art, such as described in the Examples herein.

In a preferred embodiment approximately 100 µg myosin/mouse is used for immunisation, for example 50-150 µg myosin/mouse, such as 60-140, 70-130, 80-120, 90-110 µg myosin/mouse. Preferably 100 µg myosin/mouse is used.

In one aspect the myosin is emulsified in complete Freund's adjuvant (CFA). In a further aspect Pertussis toxin is also administered to the mouse, for example one week after the immunisation with cardiac myosin. Sources of CFA and Pertussis toxin are known in the art and commercially available.

As described in the Examples below, EAM was induced in humanized DR4 mice by the injection of human cardiac α-myosin emulsified in CFA along with Pertussis toxin. CFA was applied as an adjuvant to create the bystander effect via non-specific induction of pro-inflammatory pathways. Pertussis toxin was administered as a second adjuvant enhancing the severity of the disease mainly by favouring the differentiation of autoimmune Th1 cells, hence lowering the antigenic threshold for initiation of a potential autoimmune response.

In one aspect the invention provides use of a DR4 mouse as described herein to study experimental autoimmune myocarditis or myocarditis. The DR4 mouse may be a mouse according to the invention as described herein.

In a further embodiment, the invention provides a method for producing an animal model of EAM comprising immunising a DR4 mouse with cardiac myosin. In preferred embodiments the cardiac myosin is cardiac α-myosin, more preferably human cardiac α-myosin. In one aspect the myosin is emulsified in complete Freund's adjuvant (CFA). In a preferred aspect the method comprise administering approximately 100 µg myosin/mouse. In a further preferred aspect the method also comprises administering Pertussis toxin to the said mouse. Suitable methods will be known to one skilled in the art, as described in the present Examples.

The invention will now be further described by way of Examples, which are meant to serve to assist one of ordinary skill in the art in carrying out the invention and are not intended in any way to limit the scope of the invention.

### EXAMPLES

### EXAMPLE 1

### MATERIALS AND METHODS

### Myosin extraction from human heart

A suitably sized chunk of the left ventricular wall was taken out, thawed and minced. Each gram of minced heart was homogenized in 10 ml of Solution A (0.4 M KCI; 0.15 M K2HPO4; 0.01 M Na4P2O7; 0.001 M MgCl2; 0.002 M DTT; pH:6.8 adjusted by KH2PO4; all chemicals used in this protocol were obtained from Sigma Aldrich) and centrifuged at 150,000 xg for 1 hour to clear the muscle residues and cellular debris. The supernatant was diluted with >20 volumes of 2 mM DTT to precipitate filamentous myosin which was pelleted by subsequent centrifugation at 50,000 xg for 20 mins. The pellet was re-suspended in 10 ml of Solution B (0.3 M KCI; 0.004 M MgCl2; 0.025 M imidazole; 0.01 M DTT; 0.001 M EGTA; pH:7.4 adjusted by HCI) and centrifuged at 43,000 xg for 30 mins to remove actin. The supernatant was diluted with >7 volumes of 2 mM DTT and centrifuged at 12,000 xg for 20 minutes. The pellet was re-suspended in 6 ml 0.3 M KCI, 0.01 M imidazole buffer and centrifuged at 43.000xg for 30 mins to remove actomyosin. The supernatant was again diluted with >6 volume of 2 mM DTT and the myosin precipitate was collected by centrifugation at 12,000 xg for 15 mins. The pellet was then re-dissolved in 1 ml PBS/1 g minced heart and stored at -20 °C. PMSF (0.1 mM) was added in all steps to prevent proteolysis. The purity of the cardiac myosin extracts was checked by SDS gel electrophoresis (SDS-PAGE) on a 4-20% gradient Tris-glycine gel (Farm Fresh Gels; Figure 1).

### Immunization of DR4 mice with human myosin

Appropriate amount of myosin extract was diluted to the desired volume with PBS, then mixed with equal volume of complete Freund's adjuvant (CFA; Difco) containing 8 mg/ml of heat-killed Mycobacterium Tuberculosis (hkMTB; Difco). The mixture was sonicated for 20 min, then mixed well by a 1 ml syringe until the emulsion became very thick and viscous. 100 µl of the emulsion was applied subcutaneously from tail base at a dose of 100 µg myosin/mouse. On day 0 and 2, 200 ng Pertussis toxin (Sigma Aldrich) in 0.5 ml of PBS was intraperitoneally injected to each mouse as a second adjuvant. In the first trial, the myosin and Pertussis toxin injections were repeated after a week. However, this second set of injections was excluded in later experiments to avoid induction of a too severe disease that might obscure any benefit of the peptide therapy. The experimental protocol is shown in Figure 2. Control groups received PBS in hkMTB-boosted CFA (but without human cardiac myosin) as well as Pertussis toxin. The development of EAM was assessed by echocardiographic and histopathological measurements after 3 weeks from myosin injection. Spleens were collected to measure cardiac myosin responsive T cell proliferation. Bloods were collected to measure circulating anti-myosin antibody levels.

### Echocardiographic analysis of left ventricle function

At the end of the 3 week experiment, the mice were anesthetised by intraperitoneal injection of Tribromoethanol (Avertin; SigmaAldrich) at a dose of 250 mg/kg. The left ventricle functions and diameters were analysed using Vevo 770 high-frequency, high-resolution echocardiography system (Visual Sonics) just before terminating the mice. The anesthetised mice were laid supine and butted to a heated platform by surgical tape stripes. Chest hair were removed by applying hair removal cream and Aquasonic Clear ultrasound transmission gel (Parker Laboratories) was applied to the hairless chest.

A left ventricle short-axis view at the papillary muscle level was obtained to measure the following parameters in M-Mode:
- Left ventricular end systolic diameter (LVESD)
- Left ventricular end diastolic diameter (LVEDD)
- Fractional shortening (FS = LVEDD- LVESD)
- Ejection fraction (EF)

Images were taken at a heart rate between 400-500 beats per minute. Measurements were done using Vevo 770 Software (Visual Sonics). FS is simply the difference between end-diastolic and end-systolic diameters divided by end-diastolic diameter and correlates closely to EF. EF is defined as the fraction of blood pumped out from left ventricle with each contraction.

### Histopathological analysis of myocarditis

The heart was dissected and fixed for 24 hours in 10% (v/v) formalin in PBS for histological analyses. The heart was axially cut in half to reveal both ventricles using a sharp razor blade and were then kept in PBS until processed. The tissues were then routinely processed by passing through a set of graded alcohol solutions for dehydration using Shandon Excelsior Tissue Processor (Thermo Electron Corporation).

Heart tissues were then embedded in paraffin wax using Shandon Histocentre 3 Embedding Center (Thermo Electron Corporation). The cut surface of the heart was resting on the base of the mould. The wax used was Fibro-wax (BDH Ltd) with a melting point of 56°C. The front of the plastic cassette was labelled to easily identify the tissue.

Blocks containing one half of the heart were trimmed using Shandon Finesse 325 Manual Microtome (Thermo Electron Corporation). The angle of the block in the holder was adjusted as required to obtain an appropriate orientation in relation to the blade holder. The thickness of the cut section was set to 15µm for the initial trimming. Once a complete heart section was visible, the tissue was placed on a slide and the anatomic location of the section was checked under the microscope. The thickness of the cut section was then changed to 5µm and 3 × 4 sections were collected with a 10 sections gap. Slides were stored overnight in an incubator at 37°C to dry before Haemotoxylin & Eosin (H&E) staining.

To be able to assess any potential cell infiltration, the heart sections were stained with H&E using the assay developed by Culling in 1963. Briefly, slides with mounted sections were placed on a rack and the following staining protocol was applied using Shandon Varistain 24-4 Automatic Slide Stainer (Thermo Electron Corporation);
- De-wax in clearene (Leica Biosystems) twice (3 min each)
- Transfer to 100% industrial methylated spirit (IMS; Genta Medical) twice (3 min each)
- Rehydrate in tap water (5 min)
- Stain in haematoxylin (Pioneer Research Chemicals Ltd) (2 min)
- Transfer to distilled water (3 min)
- Transfer to Scott's tap water substitute (1 min)
- Rinse in running tap water (3 min)
- Stain in 0.5% eosin (Pioneer Research Chemicals Ltd) (1.2 min)
- Rinse in running tap water (3 min)
- Dehydrate in 100% IMS (Genta Medical) twice (5 min each)
- Clear in clearene (Leica Biosystems) twice (5 min each)

The sections were mounted immediately after the staining using DPX mounting medium (VWR Ltd).

For the histopathological analysis, an Aperio slide scanner (Leica Biosystems) and the corresponding software (Aperio ImageScope) were used to capture images. All images were captured with the same objective lens (20x), which allow us to zoom in and out from 1x to 20x using the Aperio ImageScope software. The inflammatory cell infiltrate ratios were determined using ImageJ software.

### ELISA based determination of circulating anti-myosin antibody levels

A custom made enzyme-linked immonosorbent assay (ELISA) was developed to measure the level of circulating anti-myosin antibodies. Bloods were collected from the abdominal aorta under terminal anaesthesia and kept at room temperature for 30 min, then at least 2 hours on ice to allow blood sufficient time to clot until serums were separated by 15 min centrifugation at 8500 x g.

Briefly, Nunc Immuno MaxiSorp 96 well flat bottom plates were washed once with 200µL carbonate-bicarbonate buffer (pH 9.0) per well. Plates were then coated with 100µL of human cardiac myosin extract at a concentration of 10 µg/ml in 0.05M carbonate-bicarbonate buffer and incubated overnight at 4°C. The plates were then washed four times with 0.05% (v/v) Tween-20 in PBS (PBST). Non-specific binding sites were blocked by adding 100µL 2% BSA (Sigma-Aldrich) followed by incubation at 37 C° for an hour. The plates were washed twice with PBST again. For the detection of anti-myosin antibodies, serum samples were diluted 1:1000, 1:2000 and 1:4000 in diluent buffer (1 % BSA 0.01% Tween-20 in PBS). 100 µL of diluted sample was added to corresponding wells and the plates were tightly wrapped and incubated overnight at 4°C. At the end of incubation, samples were removed from the plates and the wells were washed four times with PBST. Goat anti-mouse IgG1 or IgG2c secondary antibodies conjugated to alkaline phosphatase (Abcam) were diluted 1:1000 in diluent buffer and 100µL was added to corresponding well followed by a 2 hr incubation at 37°C.

After incubation with the alkaline phosphatase-conjugated antibodies, once again the plates were washed four times with PBST. Finally, 100 µl of the p-Nitrophenyl Phosphate Liquid Substrate System (Sigma-Aldrich) was added to each well followed by 1 hour incubation at 37°C. Once the colour developed, the reaction was stopped by adding 50µL of 2M NaOH (Sigma, UK) and the absorbance was measured using LT-5000MS ELISA Reader (Labtech) at a wavelength of 405nm.

### Assessment of anti-myosin T cell proliferation levels

The splenocytes obtained from myosin immunised mice (as described herein) were cultured in 96 well plates in X-Vivo 15 media (Lonza) at a concentration of 5x105 cells/well. The cells were incubated for 72 hours at 37 C° at 5% CO2 in the presence or absence of myosin extract (at a final concentration of 20, 2 and 0.2 µg/ml). At the end of 72 hours, cells were pulsed with 3H-Tymidine for 18 hours at the same conditions by adding 25 µl of diluted 3H-Tymidine solution in X-Vivo 15 to each well (final concentration 0.5 µCi/well). Finally, the cells were kept at -20 C° at least overnight until ready to harvest.

After thawing the plates, the cells were harvested onto glass fibre filters (Cox Scientific) using Combo Cell Harvester (Skatron). Then the filters were soaked in Betaplate Scint liquid scintillation cocktail (Perkin Elmer) and sealed into sample bags (Perkin Elmer). Finally, the sealed bags containing the filters were placed into cassettes and the amount of scintillation was read using 1450 Microbeta Liquid Scintillation Counter (Perkin Elmer) in order to determine the level of 3H-tymidine incorporation.

### In silico prediction of T cell epitopes on α-myosin

Three different online MHC binding algorithms (ProPred, NetMHCII and IEDB) were used to predict the most likely T cell epitopes from α-myosin. The 15 amino acid length strong binders were calculated by these online prediction tools. Using those predictions, we defined ten peptides (15 aa in length) as the most likely T cell epitope candidates. The peptides are shown in Table 1.

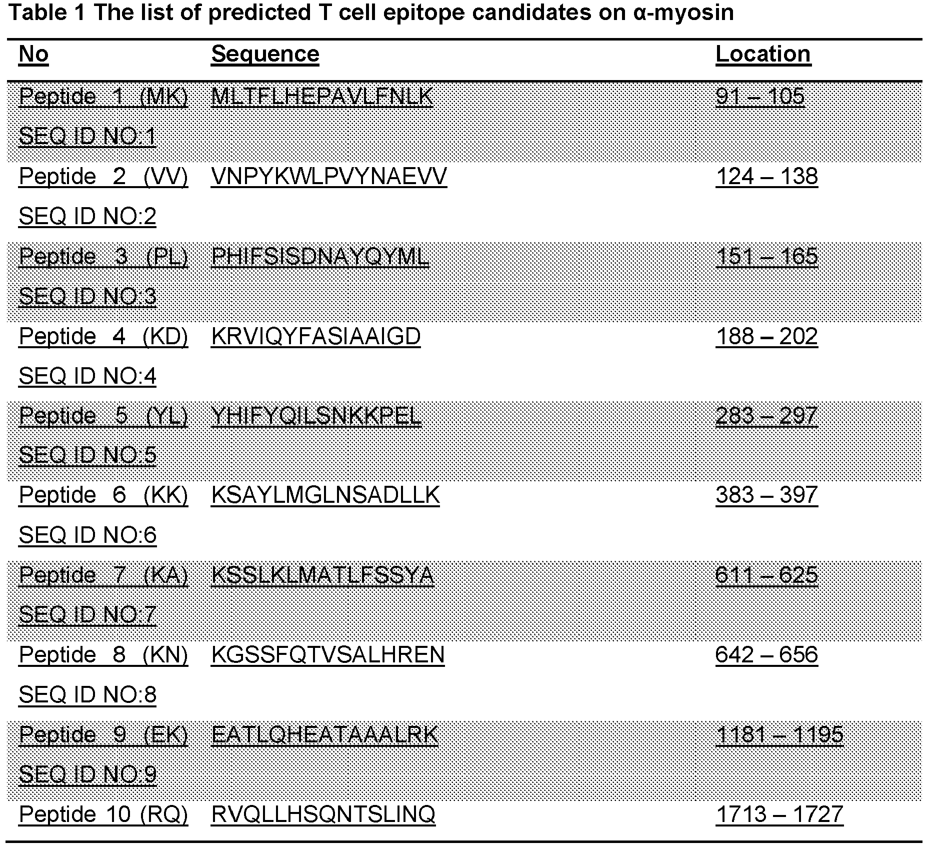

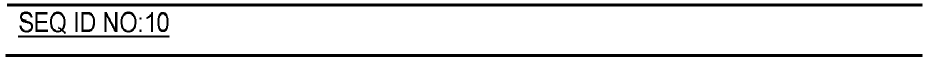

### Testing the antigenicity of T cell epitopes

The antigenicity of predicted peptides was assessed T cell proliferation against human cardiac myosin was measured by comparison of their stimulation index determined by 3H-thymidine incorporation assay (in brief: cells were pulsed with ³H-Thymidine for 18 hours at the same conditions by adding 25 µl of diluted ³H-Tymidine solution in RPMI to each well (final concentration 0.5 µCi/well). Finally, the cells were kept at -20 C° at least overnight until ready to harvest. After thawing the plates, the cells were harvested onto glass fibre filters (Cox Scientific) using a Combo Cell Harvester (Skatron). Then the filters were soaked in Betaplate Scint liquid scintillation cocktail (Perkin Elmer) and sealed into sample bags (Perkin Elmer). Finally, the sealed bags containing the filters were placed into cassettes and the amount of scintillation was read using 1450 Microbeta Liquid Scintillation Counter (Perkin Elmer) in order to determine the level of ³H-tymidine incorporation.). The α-myosin sensitized splenic cells were challenged with each peptide (GenScript (USA), peptide concentrations were 30, 3 and 0.3 µg/ml) or whole myosin (30 µg/ml).

### Cardiac myosin-specific peptide therapy protocol

2 different combinations of peptides were used for the therapy. Each cocktail contained 3 peptides chosen according to their stimulation index. The peptides were injected subcutaneously in PBS. Therapy was started with a very low dose (0.1 µg/mouse - 0.033 µg of each peptide) once mice became 6-8 weeks old. The dose was increased 10 fold every two days until the top dose of 100 µg/mouse (33.3 µg of each peptide) was reached. The top dose was repeated 3 times, then induced myocarditis with human myosin immunisation and p. toxin injections as previously described. The top dose was injected every 4 days in order to maintain the tolerance against α-myosin until the end of the protocol. The protocol is shown in Figure 9. Mice were terminated 3 weeks after the myosin injection and disease development and adaptive immune responses against cardiac myosin were assessed.

### Induction of EAM

EAM was induced in humanized DR4 mice by the injection of human cardiac α-myosin emulsified in CFA along with Pertussis toxin. CFA was applied as an adjuvant to create the bystander effect via non-specific induction of pro-inflammatory pathways. Pertussis toxin was administered as a second adjuvant enhancing the severity of the disease mainly by favouring the differentiation of autoimmune Tₕ1 cells, hence lowering the antigenic threshold for initiation of a potential autoimmune response.

In the literature, CFA and Pertussis toxin are widely applied to induce other experimental autoimmune diseases such as experimental autoimmune encephalomyelitis (EAE; and experimental autoimmune uveitis (EAU).

In our first trial, it was planned to follow disease progression using echocardiography at 3 and 6 weeks. However, sudden death occurred in 3 out of 5 mice in the α-myosin treated group after 3 weeks, whereas the animals in the control group were all healthy and no signs of myocarditis were detected in their hearts by echocardiographic measurements (data not shown). To avoid unnecessary mortality, follow up of subsequent experiments was always ended after 3 weeks.

### Increased T cell proliferation against cardiac myosin

It was checked whether an autoimmune response could be induced against cardiac myosin at the T cell level. In cultured splenocytes obtained from either α-myosin immunized or control mice in the presence of various concentrations of human cardiac myosin, the human myosin injected group showed a significant increase in T cell proliferation (5-6 fold increase) that was even stronger than the increase achieved by the mitogen, ConA, compared to the control group, which also received CFA and Pertussis toxin, but not human myosin (Figure 3). This proved that the α-myosin successfully sensitized T cell clones using our immunization protocol. Both Th1 and Th2 cell driven antibodies against α-myosin were increased during development of the disease.

### Increased circulating anti-myosin antibody levels

To check whether a humoral immune response was also induced against cardiac α-myosin in our EAM model, circulating anti-myosin antibody levels were measured. The antibody levels were measurable even at 1 in 4000 dilution in EAM induced mice, whereas the anti-myosin antibodies were undetectable in the control group. Both IgG1 (Figure 4A) and IgG2c (Figure 4B) antibodies were measured in order to implicate Th1 and Th2 cells in the production of auto-reactive antibodies.

### Decreased weight gain

Body weights of the mice were measured before the induction of EAM and at termination. There was no significant difference at the beginning. After induction of the EAM, mice almost stopped gaining weight, whereas the control group kept gaining weight as usual. At the end, the control group was significantly heavier than the diseased group (p=0.04; n=5; Figure 5). The reduction in body weight is observed in other EAM models as well.

### Impaired left ventricular cardiac function

Development of EAM was assessed by measuring the left ventricular cardiac function. A significant increase (p=0.039; n=5) was observed in systolic dimension of the diseased group as a sign of cardiac dilation (Figure 6B). Myosin inoculation along with CFA and Pertussis toxin also leaded to a significant decrease both in percentage of ejection fraction (p=0.003; n=5) and fractional shortening (p=0.006; n=5) as compared to the control group that received only CFA and Pertussis toxin (Figure 6C and D). Ejection fraction and fractional shortening are important parameters for indication of heart failure including myocarditis. These results clearly demonstrate that the myocarditis induction protocol we applied to DR4 mice lowered the cardiac performance by inducing left ventricular dysfunction.

### Inflammatory cell infiltration into cardiac tissue

To further assess the development of EAM, histopathological analysis was performed for tracking the presence of inflammatory cellular infiltrate in cardiac tissue. The increased presence of cell nuclei stained in blue, which are located in between the myocytes, represents the inflamed areas (pointed with an arrow on Figure 7). The damaged myocytes may also be seen on Figure 7. There were no sign of cell infiltration and myocyte damage in any of the mice within the control group, whereas 5 out of 5 of the mice in the EAM induced group showed some level of inflammation and tissue damage (p=0.0079, Fisher's exact test). The histopathological findings along with the echocardiographic measurements clinically proved that EAM in humanized DR4 mice was induced by administrating human cardiac α-myosin with the established protocol.

### The potentially therapeutic epitopes

We tested the binding efficiency of the candidate peptides by comparing their stimulation index on α-myosin-sensitized splenic cells obtained from EAM induced DR4 mice (the myosin group in red on Figure 8) with the stimulation obtained on splenic cells collected from the mice not injected with myosin, but with CFA and Pertussis toxin (the control group in orange on Figure 8). We repeated the screening assay three times and the best inducers were selected as the potentially therapeutic dominant epitopes. We picked 6 peptides in total and made two groups (circled in red and purple on Figure 8) to be applied in peptide therapies. YL (SEQ ID NO:5) gave consistently the strongest stimulation amongst the peptides (sometimes even more than whole myosin protein). PL (SEQ ID NO:3) and VV (SEQ ID NO:2) were the other two peptides giving stronger stimulation than the rest of them. That is why we first started using the combination of these peptides (YL, PL and VV). RQ (SEQ ID NO:10), EK (SEQ ID NO:9) and KA (SEQ ID NO:7) were selected as an alternative combination to be used in therapies. MK was another peptide with a relatively good stimulation index. However, due to solubility issues, we excluded it from our selection to avoid any potential precipitation during preparation of the peptide cocktails.

The overall efficacy was lower in the second screening assay. Whole myosin gave only a 2 fold stimulation, but YL and PL peptides provided stimulation even at the lowest concentration (0.3 µg/ml).

### Effect of cardiac myosin-specific immunotherapy

the selected peptide combinations were applied in a dose escalation regime (Figure 9) through a subcutaneous route and assessed both immunological and clinical effects of the therapy on the development of EAM by measuring cardiac α-myosin-specific T cell proliferation and antibody levels as well as left ventricle cardiac function and inflammatory infiltrate using our DR4 mouse EAM model.

### Decreased T cell proliferation against cardiac myosin

It was possible to downregulate the auto-reactive T cell response against cardiac myosin with the peptide therapy. While the decrease in myosin responsive T cell proliferation was very significant (p=0.0007; n=15) in the group treated with YL-PL-VV peptides compared to the control group without the therapy (from 5.6 fold to 3.1 fold), it was only a tendency at borderline significance (p=0.065; n=11) with the RQ-EK-KA peptides (Figure 10).

### Decreased circulating anti-myosin antibody levels

Anti-myosin IgG1 and IgG2c antibodies were decreased significantly, compared to the control group, with the therapy performed with RQ-EK-KA peptides (For IgG1 p=0.036 in 1/4000 dilution, p=0.019 in 1/2000 dilution, p=0.011 in 1/1000 dilution; For IgG2c p=0.0072 in 1/1000 dilution; n=11; Figure 11). The reducing effect of YL-PL-VV therapy on α-myosin-specific autoimmunity may be limited to the T cell responses.

### Improved left ventricular cardiac function

Although, no effect of the therapies on either systolic or diastolic dimensions of the left ventricle was observed (Figure 12A), both YL-PL-VV and RQ-EK-VV therapies significantly improved the left ventricular cardiac function by increasing the percentages of ejection fraction (p=0.011, n=10 for RQ-EK-KA therapy; p=0.041, n=13 for YL-PL-VV therapy) and fractional shortening (p=0.011, n=10 for RQ-EK-KA therapy; p=0.044, n=13 for YL-PL-VV therapy) in the EAM induced DR4 mice (Figure 12B). These results provided the first functional evidence demonstrating that a tolerogenic peptide therapy can retard the development of EAM.

### Decreased inflammatory cell infiltration into cardiac tissue

The level of cardiac inflammation was measured by looking at the percentage of immune cell infiltration into heart tissue, which can be seen as the blue areas generated by concentrated presence of inter-myocyte cell nuclei on H&E stained heart sections (Figure 13B, C and D). We observed a significant reduction (p=0.021; n=11) in cell infiltrate ratios in hearts of EAM induced mice receiving RQ-EK-YL therapy (Figure 13A). This finding provided supporting histological evidence suggesting that the induction of tolerance against α-myosin by peptide therapy can potentially be an effective treatment for EAM.

All publications mentioned in the above specification are herein incorporated by reference. Various modifications and variations of the described methods and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology, immunology or related fields are intended to be within the scope of the following claims.

The invention also relates to the following aspects as defined in the following numbered paragraphs:
1. A peptide comprising:
   (i) all or a part of an amino acid sequence selected from:
      MLTFLHEPAVLFNLK (SEQ ID NO:1),
      VNPYKWLPVYNAEVV (SEQ ID NO:2),
      PHIFSISDNAYQYML (SEQ ID NO:3),
      KRVIQYFASIAAIGD (SEQ ID NO:4),
      YHlFYQlLSNKKPEL (SEQ ID NO:5),
      KSAYLMGLNSADLLK (SEQ ID NO:6),
      KSSLKLMATLFSSYA (SEQ ID NO:7),
      KGSSFQTVSALHREN (SEQ ID NO:8),
      EATLQHEATAAALRK (SEQ ID NO:9) and
      RVQLLHSQNTSLINQ (SEQ ID NO:10); or
   (ii) an amino acid sequence having at least 60% sequence identity to the sequences of any of SEQ ID NO:1 to 10.
2. A peptide according to paragraph 1, which is selected from SEQ ID NOs:1 to 10.
3. A composition comprising one or more peptides as defined in paragraph 1 or paragraph 2.
4. A composition according to paragraph 3 which comprises peptides of SEQ ID NOs:2, 3 and 5.
5. A composition according to paragraph 3 which comprises peptides of SEQ ID NOs:7, 9 and 10.
6. A peptide according to paragraph 1 or paragraph 2, or a composition according to any one of paragraphs 3 to 5 for use in supressing or preventing the production of α-myosin autoantibodies in a subject.
7. A peptide according to paragraph 1 or paragraph 2, or a composition according to any one of paragraphs 3 to 5 for use in treating and/or preventing myocarditis in a subject.
8. The use of a peptide according to paragraph 1 or paragraph 2, or a composition according to any one of paragraphs 3 to 5 in the manufacture of a medicament to suppress or prevent the production of α-myosin autoantibodies in a subject.
9. The use of a peptide according to paragraph 1 or paragraph 2, or a composition according to any one of paragraphs 3 to 5 in the manufacture of a medicament to treat and/or prevent myocarditis in a subject.
10. A method for supressing or preventing the production of α-myosin autoantibodies in a subject, which comprises the step of administration of a peptide according to paragraph 1 or paragraph 2, or a composition according to any one of paragraphs 3 to 5, to the subject.
11. A method for treating myocarditis in a subject which comprises the step of administration of a peptide according to paragraph 1 or paragraph 2, or a composition according to any one of paragraphs 3 to 5, to the subject.
12. A peptide or composition for use, use or method according to any one of paragraphs 6 to 11 wherein the myocarditis is dilated cardiomyopathy.
13. A peptide or composition for use, use or method according to any one of paragraphs 6 to 11, wherein the subject is HLA-DR4 positive.
14. A nucleic acid molecule encoding a peptide according to paragraph 1 or paragraph 2.
15. A mouse model of experimental autoimmune myocarditis comprising an HLA-DR4tg mouse which has been immunised with cardiac myosin.

## Claims

1. A peptide comprising:
(i) all or a part of an amino acid sequence selected from:
MLTFLHEPAVLFNLK (SEQ ID NO:1),
VNPYKWLPVYNAEVV (SEQ ID NO:2),
PHIFSISDNAYQYML (SEQ ID NO:3),
KRVIQYFASIAAIGD (SEQ ID NO:4),
YHIFYQILSNKKPEL (SEQ ID NO:5),
KSAYLMGLNSADLLK (SEQ ID NO:6),
KSSLKLMATLFSSYA (SEQ ID NO:7),
KGSSFQTVSALHREN (SEQ ID NO:8),
EATLQHEATAAALRK (SEQ ID NO:9) and
RVQLLHSQNTSLINQ (SEQ ID NO:10); or
(ii) an amino acid sequence having at least 60% sequence identity to the sequences of any of SEQ ID NO:1 to 10.

2. A peptide according to claim 1, which is selected from SEQ ID NOs:1 to 10.

3. A composition comprising one or more peptides as defined in claim 1 or claim 2.

4. A composition according to claim 3 which comprises peptides of SEQ ID NOs:2, 3 and 5.

5. A composition according to claim 3 which comprises peptides of SEQ ID NOs:7, 9 and 10.

6. A peptide according to claim 1 or claim 2, or a composition according to any one of claims 3 to 5 for use in supressing or preventing the production of α-myosin autoantibodies in a subject.

7. A peptide according to claim 1 or claim 2, or a composition according to any one of claims 3 to 5 for use in treating and/or preventing myocarditis in a subject.

8. The use of a peptide according to claim 1 or claim 2, or a composition according to any one of claims 3 to 5 in the manufacture of a medicament to suppress or prevent the production of α-myosin autoantibodies in a subject.

9. The use of a peptide according to claim 1 or claim 2, or a composition according to any one of claims 3 to 5 in the manufacture of a medicament to treat and/or prevent myocarditis in a subject.

10. A method for supressing or preventing the production of α-myosin autoantibodies in a subject, which comprises the step of administration of a peptide according to claim 1 or claim 2, or a composition according to any one of claims 3 to 5, to the subject.

11. A method for treating myocarditis in a subject which comprises the step of administration of a peptide according to claim 1 or claim 2, or a composition according to any one of claims 3 to 5, to the subject.

12. A peptide or composition for use, use or method according to any one of claims 6 to 11 wherein the myocarditis is dilated cardiomyopathy.

13. A peptide or composition for use, use or method according to any one of claims 6 to 11, wherein the subject is HLA-DR4 positive.

14. A nucleic acid molecule encoding a peptide according to claim 1 or claim 2.

15. A mouse model of experimental autoimmune myocarditis comprising an HLA-DR4tg mouse which has been immunised with cardiac myosin.
